# EUROPÄISCHE PATENTSCHRIFT

(11) **EP 0 548 763 B1**
(45) Veröffentlichungstag und Bekanntmachung des Hinweises auf die Patenterteilung: **29.05.1996**
(21) Anmeldenummer: 92121356.7
(22) Anmeldetag: 16.12.1992
(51) Int. Cl.: C07C 35/08, C07C 29/09

(54) **Verfahren zur Herstellung von Cycloalkanolen**
Process for the preparation of cycloalcanols
Procédé de préparation de cycloalcanols

(30) Priorität: 24.12.1991 DE 4142944
(43) Veröffentlichungstag der Anmeldung: 30.06.1993
(73) Patentinhaber: BASF Aktiengesellschaft, D-67063 Ludwigshafen (DE)
(72) Erfinder: Krug, Thomas, W-6710 Frankenthal (DE); Fischer, Rolf, Dr., W-6900 Heidelberg (DE); Pinkos, Rolf, Dr., W-6702 Bad Duerkheim (DE)

(56) Entgegenhaltungen:
- EP-A- 0 341 163
- EP-A- 0 476 400

## Beschreibung

Gegenstand der Erfindung ist ein Verfahren zur Herstellung von Cycloalkanolen durch Hydrolyse von Cycloalkyl-C₁-C₄-fettsäureestern.

Aus der NL-A-70 04 497 ist bekannt, daß man aus Cyclohexylester durch alkalische Hydrolyse Cyclohexanol gewinnt. Nachteilig ist, wie bei der alkalischen Verseifung generell, daß die Carbonsäure als Salz anfällt und somit aufwendig zurück gewonnen werden muß. Ferner hat das Verfahren den Nachteil, daß erhebliche Mengen an behandlungsbedürftigen Abwässern anfallen.

Nach einem anderen in der polnischen Patentschrift 63 551 (Chemical Abstracts, Vol. 76, Ref. 126476x) beschriebenen Verfahren werden Cyclohexylester unter Mitverwendung von Salpetersäure oder Schwefelsäure gespalten. Das Cyclohexanol wird durch eine energieaufwendige Wasserdampfdestillation gewonnen.

Es war deshalb die technische Aufgabe gestellt, ein Verfahren zur Herstellung von Cycloalkanolen durch Hydrolyse von Cycloalkyl-C₁-C₄-fettsäureestern zur Verfügung zu stellen, bei dem eine hohe Selektivität hinsichtlich der Cycloalkanole und Fettsäuren erzielt wird, wenig Cycloalken als Nebenprodukt anfällt, die Säuren leicht zurückzugewinnen sind und möglichst kein behandlungsbedürftiges Abwasser anfällt.

Diese Aufgabe wird gelöst in einem Verfahren zur Herstellung von Cycloalkanolen durch Hydrolyse von Cycloalkyl-C₁-C₄-fettsäureestern, welches folgende Schritte umfaßt:
a) Umsetzen von Cycloalkyl-C₁-C₄-fettsäureestern mit Wasser bei einer Temperatur von 30 bis 250°C in flüssiger Phase unter Erhalt eines Reaktionsgemisches aus Cycloalkyl-C₁-C₄-fettsäureestern, Cycloalkanol, C₁-C₄-Fettsäuren und Wasser.
b) Trennen des in Stufe a) erhaltenen Reaktionsgemisches bei einer Temperatur von 0 bis 200°C in eine obere, im wesentlichen aus Cycloalkanol und Cycloalkyl-C₁-C₄-fettsäureester bestehende Phase und eine untere, im wesentlichen aus Wasser und C₁-C₄-Fettsäuren bestehende Phase.
c) Abtrennen von Cycloalkanol aus der oberen Phase aus Stufe b) durch Destillation und Zurückführen von nicht umgesetzten Cycloalkyl-C₁-C₄-fettsäureestern in die Stufe a).

Das neue Verfahren hat den Vorteil, daß es in hoher Selektivität zu Cycloalkanol verläuft und wenig Cycloalken gebildet wird. Ferner hat das neue Verfahren den Vorteil, daß es unschwer kontinuierlich im technischen Maßstab durchführbar ist und wenig behandlungsbedürftiges Abwasser anfällt.

In einem ersten Schritt a) werden Cycloalkyl-C₁-C₄-fettsäureester mit Wasser bei einer Temperatur von 30 bis 250°C in flüssiger Phase unter Erhalt eines Reaktionsgemisches im wesentlichen aus Cycloalkyl-C₁-C₄-fettsäureester, Cycloalkanol, Fettsäure und Wasser, umgesetzt.

Bevorzugt geht man von C₁-C₄-Fettsäureestern von Cycloalkanolen mit 5 bis 8 Kohlenstoffatomen im Ring aus. Insbesondere von deren Fettsäureestern mit 1 oder 2 Kohlenstoffatomen. Besondere technische Bedeutung hat Cyclohexylformiat oder -acetat erlangt. Geeignete Cycloalkyl-C₁-C₄-fettsäureester sind z.B. Cyclopentylformiat, Cyclohexylformiat, Cyclooctylformiat, Cyclopentylacetat, Cyclohexylpropionat, Cyclooctylacetat. Die Verwendung von Cyclohexylformiat hat sich besonders bewährt. Es versteht sich, daß aus den bevorzugten Cycloalkylfettsäureestern die bevorzugten Cycloalkanole erhalten werden.

In der Regel verwendet man je Mol Cycloalkyl-C₁-C₄-fettsäureester, 0,1 bis 100 Mol Wasser, vorteilhaft 1 bis 30, insbesondere 2 bis 10 Mol Wasser.

Die Umsetzung wird bei einer Temperatur von 30 bis 250°C durchgeführt. Vorteilhaft hält man eine Temperatur von 60 bis 200°C, insbesondere 70 bis 160°C ein. Es ist möglich, die Umsetzung bei Normaldruck, erhöhtem Druck oder leichtem Unterdruck durchzuführen. Z.B. unter einem Druck von 0,3 bis 20 bar, insbesondere 0,8 bis 10 bar. Die Druck- und Temperaturbedingungen werden so gewählt, daß die Ausgangs- und Endstoffe in flüssiger Phase vorliegen.

In der Regel hält man in der Stufe a) eine Verweilzeit von 5 Minuten bis 300 Minuten ein. Bewährt haben sich Verweilzeiten von 15 Minuten bis 120 Minuten.

Die Hydrolyse der Cycloalkyl-C₁-C₄-fettsäureester kann autokatalytisch oder unter Zusatz eines sauren Katalysators durchgeführt werden. Vorteilhaft wird die Umsetzung unter Mitverwendung eines stark sauren Katalysators durchgeführt. Als saure Katalysatoren eignen sich Zeolithe, saure Ionenaustauscher, Heteropolysäuren, saure und supersaure Metalloxide, die gegebenenfalls mit Anionen wie SO₄²⁻ dotiert sind und anorganische oder organische Säuren.

Bevorzugte Zeolithe sind solche aus der Mordenitgruppe oder engporige Zeolithe vom Erionit-, Chabasit- oder Faujasit-Typ, z.B. X-, Y- oder L-Zeolithe. Ferner sind geeignet ultrastabile Zeolithe aus der Faujasitgruppe, die dealuminiert sind.

Besonders bevorzugt sind Zeolithe mit Pentasilstruktur, wie ZSM 5, ZSM 11 und ZBM 10. Diese haben als Grundbaustein einen aus SiO₂-Tetraedern aufgebauten Fünfring gemeinsam, weisen ein hohes Verhältnis von Siliciumdioxid zu Aluminiumoxid auf und haben Porengrößen, die zwischen denen der Zeolithe vom Typ A und denen vom Typ X oder Y liegen. Es haben sich Zeolithe bewährt, bei denen das Verhältnis von Siliciumdioxid zu Aluminiumoxid kleiner 100 beträgt. Die Zeolithe können vollständig oder aber auch nur teilweise in der H-Form vorliegen.

Bevorzugte saure Ionentauscher sind vernetzte Polystyrole mit Sulfonsäuregruppen oder Polyperfluoralkylsulfonsäuren.

Bevorzugte Heteropolysäuren sind Polysäuren von Molybdän oder Wolfram mit Phosphorsäure, Tellursäure, Selensäure, Arsensäure, Kieselsäure oder insbesondere mit Phosphorsäure. Als Beispiele seien genannt: Dodecawolframatophosphorsäure oder Dodecamolybdatophosphorsäure. Die Protonen der Heteropolysäuren können teilweise durch Metallionen ersetzt sein. Bevorzugt sind dabei Alkali- und Erdalkalimetallionen.

Geeignete saure Metalloxide sind beispielsweise Siliciumdioxid, Aluminiumoxid, Zirkondioxid, Titandioxid oder Zinndioxid. Zur Erhöhung der Säurestärke können solche Metalloxide vor ihrer Verwendung z.B. mit Schwefelsäure behandelt werden.

Geeignete Säuren sind beispielsweise Mineralsäuren wie Schwefelsäure oder Phosphorsäure, ferner organische Säuren wie Sulfonsäuren oder Carbonsäuren. Bevorzugt werden die Fettsäuren des eingesetzten Esters verwendet, insbesondere Ameisensäure.

Das Gewichtsverhältnis von eingesetztem Katalysator zu cyclischem Ester beträgt in der Regel von 0 bis 20, insbesondere 0 bis 5, besonders bevorzugt von 0 bis 1.

Die verwendeten Cycloalkyl-C₁-C₄-fettsäureester können bereits das entsprechende Cycloalkanol als Beiprodukt enthalten. Z.B. bis zu 50 Mol-%, insbesondere bis zu 10 Mol-%, vorteilhaft jedoch bis zu 5 Mol-%.

Man erhält ein Reaktionsgemisch, das im wesentlichen aus Cycloalkanol, Cycloalkyl C₁-C₄-fettsäureestern, C₁-C₄-fettsäuren und Wasser besteht und das ggf. geringe Mengen Cyclohexen enthalten kann.

In Schritt b) wird das in Stufe a) erhaltene Reaktionsgemisch bei einer Temperatur von 0 bis 200°C gegebenenfalls unter erhöhtem Druck, z.B. von 1 bis 10 bar, insbesondere 1 bis 5 bar, in eine obere, im wesentlichen aus Cycloalkanol und Cycloalkyl-C₁-C₄-fettsäureester bestehende Phase und eine untere, im wesentlichen aus Wasser und Fettsäure bestehende Phase getrennt.

Zur Ausbildung der Phasen hält man vorteilhaft eine Temperatur von 20 bis 140°C ein. Die Trennung der Phasen erfolgt zum Beispiel in einem Phasenscheider. Es hat sich als vorteilhaft erwiesen, zur Unterstützung der Phasentrennung Wasser zuzusetzen, z.B. 5 bis 20 Gew.-%, bezogen auf das zu trennende Gemisch.

Die obere Phase besteht im wesentlichen aus Cycloalkanol und Cycloalkyl-C₁-C₄-fettsäureester und enthält neben kleineren Mengen an Wasser, C₁-C₄-Fettsäure und Cyclohexen als Nebenprodukt. Die untere Phase besteht im wesentlichen aus Wasser und C₁-C₄-Fettsäuren und enthält daneben kleine Mengen an Cycloalkanol und Cycloalkyl-C₁-C₄-fettsäureester.

Aus der oberen Phase wird Cycloalkanol in der Regel durch Destillation abgetrennt. Vorteilhaft behandelt man die obere Phase mit Wasser, um Säuren zu entfernen. Die weitere Aufarbeitung erfolgt vorteilhaft durch Destillation, z.B. in einer Kolonne mit 20 bis 30°C theoretischen Böden. Hierbei werden in der Regel Wasser und Reste von Fettsäuren über Kopf abgetrennt, ein Gemisch aus Cycloalkanol und Cycloalkylfettsäureester als Azeotrop über einen Seitenabzug entnommen und reines Cycloalkanol am Sumpf abgezogen. Verzichtet man auf einen Seitenabzug, so erhält man in der Regel ein Kopfprodukt aus Wasser, Fettsäure, Cycloalkanol und Cycloalkylester, das in den vorgeschalteten Phasenscheider zurückgeführt werden kann, während reines Cycloalkanol im Sumpf anfüllt.

Die untere Phase, die im wesentlichen aus Wasser und Fettsäure besteht, wird zweckmäßig extrahiert nach einem Verfahren, wie es beispielsweise in den US-Patentschriften 4 642 166 oder 4 786 370 beschrieben wird. Das hierbei anfallende Wasser wird zweckmäßig wieder in die Hydrolysestufe a) zurückgeführt, während Fettsäuren zur Herstellung von Cyclohexylestern verwendet werden können.

Die nach dem Verfahren der Erfindung erhältlichen Cycloalkanole, z.B. Cyclohexanol, sind wertvolle Ausgangsstoffe für Faser-Vorprodukte.

Das Verfahren nach der Erfindung sei anhand folgender Beispiele näher erläutert:

### Beispiel 1

1,29 g Cyclohexylformiat und 0,85 g Wasser wurden in einem 25 ml Druckgefäß unter Rühren 2 h auf 150°C erhitzt. Der Druck betrug 3,2 bar. Sowohl während als auch nach der Reaktion war die Reaktionsmischung zweiphasig. Für analytische Zwecke wurde der zweiphasige Reaktionsaustrag mit Aceton homogenisiert. Es fanden sich neben unumgesetztem Cyclohexylformiat 66,5 Mol-% Cyclohexanol und 0,6 Mol-% Cyclohexen. Andere Nebenprodukte wurden nicht gefunden. Die quantitative Analyse erfolgte über GC (innerer Standard).

### Beispiel 2

Analog Beispiel 1 wurde die gleiche Reaktionsmischung unter Zusatz von 0,23 g Ameisensäure bei 110°C 2 h gerührt. Nach Homogenisieren des zweiphasigen Austrages fanden sich neben unumgesetztem Cyclohexylformiat 54 Mol-% Cyclohexanol. Nebenprodukte ließen sich nicht nachweisen.

### Beispiel 3

Analog Beispiel 1 wurden 1,28 g Cyclohexylformiat, 0,85 g Wasser und 0,1 g Molybdatophosphorsäure 30 Minuten bei 90°C umgesetzt.

Nach Homogenisieren fanden sich neben unumgesetztem Cyclohexylformiat 62,7 Mol-% Cyclohexanol. Als einziges Nebenprodukt entstand mit 0,1 Mol-% Cyclohexen.

### Beispiel 4

Analog Beispiel 3 wurden bei gleicher Vorgehensweise, aber bei 110°C 61,9 Mol-% Cyclohexanol gefunden. Einziges Nebenprodukt war Cyclohexen mit 0,7 %.

### Beispiel 5

Analog Beispiel 1 wurden 1,28 g Cyclohexylformiat, 2,70 g Wasser und 0,5 g sulfoniertes Polytetrafluorethylen 2 h auf 110°C erhitzt. Nach Homogenisieren der flüssigen Phase fanden sich neben unumgesetztem Cyclohexylformiat 75,8 Mol-% Cyclohexanol. Als einziges Nebenprodukt entstand Cyclohexen mit 0,9 Mol-%.

### Beispiel 6

Analog Beispiel 1 wurden 1,28 g Cyclohexylformiat, 1,8 g Wasser und 0,5 g eines sulfonierten Polytetrafluorethylens 2 h auf 110°C erhitzt. Nach Abtrennen des Katalysators und Homogenisieren mit Aceton fanden sich neben unumgesetztem Cyclohexylformiat 70,2 Mol-% Cyclohexanol und als einziges Nebenprodukt 1,8 Mol-% Cyclohexen.

### Beispiel 7

Analog Beispiel 1 wurden 7,6 g Wasser, 0,2 g Ameisensäure, 1,4 g Cyclohexanol und 10,8 g Cyclohexylformiat 2 h bei 130°C umgesetzt. Der zweiphasige Austrag wurde in einem Phasenabscheider in die einzelnen Phasen aufgetrennt.

In der oberen Phase fanden sich 0,02 g Cyclohexen, 6,90 g Cyclohexanol, 3,50 g Cyclohexylformiat, 1,3 bis 1,4 g Ameisensäure und ca. 1 g Wasser (der Ameisensäuregehalt wurde potentiometrisch, der Wassergehalt nach Karl-Fischer bestimmt).

In der unteren Phase fanden sich 0,20 g Cyclohexanol, 0,02 g Cyclohexylformiat, 1,4 bis 1,5 g Ameisensäure und ca. 5 g Wasser.

### Beispiel 8

Analog Beispiel 1 wurden 1,8 g Cyclohexylformiat, 3,57 g Wasser und 0,11 g Phosphorsäure (85 %ig) zwei Stunden auf 110°C erhitzt. Der zweiphasige Austrag wurde in die einzelnen Phasen aufgeteilt. In der oberen Phase (3,80 g) fanden sich 75,3 Mol-% Cyclohexanol und 18,2 Mol-% Cyclohexylformiat. In der unteren Phase (1,68 g) fand sich 6,5 Mol-% Cyclohexanol. Cyclohexen wurde nicht gefunden.

### Beispiel 9

Analog Beispiel 1 wurden 2,0 g Cyclohexylacetat, 1,16 g Wasser und 0,5 g vernetztes Polystyrol mit Sulfonsäuregruppen 30 Minuten bei 120°C umgesetzt. Der zweiphasige Reaktionsaustrag wurde homogenisiert. Neben unumgesetztem Cyclohexylacetat wurden 16,7 Mol-% Cyclohexanol gefunden. Einziges Nebenprodukt war Cyclohexen mit 0,6 Mol-%.

### Beispiel 10

Analog Beispiel 1 wurden 115 g Cyclohexylformiat, 5 g Molybdatophosphorsäure und 240 g Wasser 1 Stunde bei 90°C umgesetzt. Nach Abkühlen des Reaktionsgemisches auf 25°C wurde die obere Phase (105 g) in eine Destillationsapparatur überführt. Bei 1013 mbar und 170°C Sumpftemperatur destillierten 50 g eines zweiphasigen Gemisches ab (15 Gew.% Cyclohexanol, 66 Gew.% Cyclohexylformiat, 2 Gew.% Cyclohexen, 17 Gew.% Wasser und Ameisensäure). Als Rückstand verblieben 55 g. Nach GC enthielt der Rückstand 98 % Cyclohexanol (60 % Ausbeute). Die untere Phase bestand im wesentlichen aus Wasser, Ameisensäure und Katalysator.

## Patentansprüche

1. Verfahren zur Herstellung von Cycloalkanolen durch Hydrolyse von Cycloalkyl-C₁-C₄-fettsäureestern, welches folgende Schritte umfaßt:
a) Umsetzen von Cycloalkyl-C₁-C₄-fettsäureestern mit Wasser bei einer Temperatur von 30 bis 250°C in flüssiger Phase unter erhalt eines Reaktionsgemisches im wesentlichen aus Cycloalkyl-C₁-C₄-fettsäureestern, Cycloalkanol, Fettsäuren und Wasser.
b) Trennen des in Stufe a) erhaltenen Reaktionsgemisches bei einer Temperatur von 0 bis 200°C in eine obere, im wesentlichen aus Cycloalkanol und Cycloalkyl-C₁-C₄-fettsäureester bestehenden Phase und in eine untere, im wesentlichen aus Wasser und C₁-C₄-Fettsäuren bestehende Phase.
c) Abtrennen von Cycloalkanol aus der oberen Phase aus Stufe b) durch Destillation und Zurückführen von nicht umgesetzten Cycloalkyl-C₁-C₄-fettsäureestern in die Stufe a).

2. Verfahren nach Anspruch 1, dadurch gekennzeichnet, daß je Mol Cycloalkyl-C₁-C₄-fettsäureester 1 bis 30 Mol Wasser mitverwendet.

3. Verfahren nach den Ansprüchen 1 und 2, dadurch gekennzeichnet, daß man Zeolithe, saure Ionenaustauscher, Heteropolysäuren, saure Metalloxide und Mineralsäuren oder organische Carbon- oder Sulfonsäuren als Katalysatoren mitverwendet.

4. Verfahren nach den Ansprüchen 1 bis 3, dadurch gekennzeichnet, daß man in Stufe a) eine Temperatur von 70 bis 160°C einhält.

5. Verfahren nach den Ansprüchen 1 bis 4, dadurch gekennzeichnet, daß man in Stufe a) eine Verweilzeit von 15 bis 120 Minuten einhält.

6. Verfahren nach den Ansprüchen 1 bis 5, dadurch gekennzeichnet, daß man in der Stufe b) bei der Phasentrennung eine Temperatur von 20 bis 140°C unter einem Druck von 1 bis 5 bar einhält.

7. Verfahren nach den Ansprüchen 1 bis 6, dadurch gekennzeichnet, daß man in der Stufe b) zur Phasentrennung zusätzlich Wasser zusetzt.

8. Verfahren nach den Ansprüchen 1 bis 7, dadurch gekennzeichnet, daß man die obere Phase aus der Stufe b) in einer Kolonne destilliert, am Kopf ein Gemisch aus Fettsäure und Wasser abnimmt, im Verlauf der Kolonne im mittleren Teil ein Azeotrop aus Cycloalkanol und Cycloalkyl-C₁-C₄-fettsäureester entnimmt und im Sumpf Cycloalkanol abzieht.

9. Verfahren nach den Ansprüchen 1 bis 8, dadurch gekennzeichnet, daß man von Cyclohexyl-C₁- oder -C₂-fettsäureestern ausgeht.

## Claims

1. A process for preparing cycloalkanols by hydrolysis of cycloalkyl C₁-C₄ fatty acid esters, which comprises the following steps:
a) reacting cycloalkyl C₁-C₄ fatty acid esters with water at a temperature from 30 to 250°C in liquid phase to obtain a reaction mixture essentially of cycloalkyl C₁-C₄ fatty acid esters, cycloalkanol, fatty acids and water,
b) separating the reaction mixture obtained in stage a) at a temperature from 0 to 200°C into an upper phase, consisting essentially of cycloalkanol and cycloalkyl C₁-C₄ fatty acid esters, and a lower phase, consisting essentially of water and C₁-C₄ fatty acids,
c) removing cycloalkanol from the upper phase of stage b) by distillation and recycling unconverted cycloalkyl C₁-C₄ fatty acid esters into stage a).

2. A process as claimed in claim 1, characterized in that 1 to 30 mol of water are used per mole of cycloalkyl C₁-C₄ fatty acid ester.

3. A process as claimed in claims 1 and 2, characterized in that zeolites, acidic ion exchangers, heteropolyacids, acidic metal oxides and mineral acids or organic carboxylic or sulfonic acids are used as catalysts.

4. A process as claimed in any of claims 1 to 3, characterized in that a temperature from 70 to 160°C is maintained in stage a).

5. A process as claimed in any of claims 1 to 4, characterized in that a residence time from 15 to 120 minutes is maintained in stage a).

6. A process as claimed in any of claims 1 to 5, characterized in that the phase separation of stage b) is carried out at a temperature from 20 to 140°C and a pressure from 1 to 5 bar.

7. A process as claimed in any of claims 1 to 6, characterized in that water is additionally added in stage b) for phase separation.

8. A process as claimed in any of claims 1 to 7, characterized in that the upper phase obtained from stage b) is distilled in a column, a mixture of fatty acid and water is taken off at the top, an azeotrope of cycloalkanol and cycloalkyl C₁-C₄ fatty acid ester is removed mid-column, and cycloalkanol is withdrawn as bottom product.

9. A process as claimed in any of claims 1 to 8, characterized in that cyclohexyl C₁ or C₂ fatty acid esters are used as starting materials.

## Revendications

1. Procédé de préparation de cycloalcanols par hydrolyse d'esters de cycloalkyle et d'acide gras en C₁-C₄, comprenant les étapes suivantes:
a) Mise en réaction d'esters de cycloalkyle et d'acide gras en C₁-C₄ avec de l'eau à une température de 30 à 250°C, en phase liquide, pour obtenir un mélange réactionnel composé essentiellement d'esters de cycloalkyle et d'acide gras en C₁-C₄, de cycloalcanol, d'acides gras et d'eau.
b) Séparation du mélange réactionnel obtenu dans l'étape a), à une température de 0 à 200°C, en une phase supérieure composée essentiellement de cycloalcanol et d'ester de cycloalkyle et d'acide gras en C₁-C₄ et en une phase inférieure composée essentiellement d'eau et d'acide gras en C₁-C₄.
c) Séparation du cycloalcanol de la phase supérieure de l'étape b) par distillation et renvoi, dans l'étape a), des esters de cycloalkyle et d'acide gras en C₁-C₄ n'ayant pas réagi.

2. Procédé selon la revendication 1, caractérisé en ce que l'on utilise simultanément, par mole d'ester de cycloalkyle et d'acide gras en C₁-C₄, 1 à 30 moles d'eau.

3. Procédé selon la revendication 1 ou 2, caractérisé en ce que l'on utilise simultanément, comme catalyseurs, des zéolithes, des échangeurs d'ions acides, des hétéropolyacides, des oxydes métalliques acides et des acides minéraux ou des acides organiques carboxyliques ou sulfoniques.

4. Procédé selon l'une quelconque des revendications 1 à 3, caractérisé en ce que l'on maintient, dans l'étape a), une température de 70 à 160°C.

5. Procédé selon l'une quelconque des revendications 1 à 4, caractérisé en ce que l'on maintient, dans l'étape a), une durée de séjour de 15 à 120 min.

6. Procédé selon l'une quelconque des revendications 1 à 5, caractérisé en ce que l'on maintient, dans l'étape b) lors de la séparation de phases, une température de 20 à 140°C sous une pression de 1 à 5 bar.

7. Procédé selon l'une quelconque des revendications 1 à 6, caractérisé en ce que l'on ajoute en plus de l'eau dans l'étape b) pour la séparation de phases.

8. Procédé selon l'une quelconque des revendications 1 à 7, caractérisé en ce que l'on distille dans une colonne la phase supérieure obtenue dans l'étape b), on extrait en tête un mélange d'acide gras et d'eau, on prélève dans la partie moyenne de la colonne un azéotrope de cycloalcanol et d'ester de cycloalkyle et d'acide gras en C₁-C₄ et on soutire en bas de colonne du cycloalcanol.

9. Procédé selon l'une quelconque des revendications 1 à 8, caractérisé en ce que l'on part d'esters de cyclohexyle et d'acide gras en C₁ ou en C₂.
